# EUROPEAN PATENT SPECIFICATION

(11) **EP 1 750 556 B1**
(45) Date of publication and mention of the grant of the patent: **13.08.2008**
(21) Application number: 05738860.5
(22) Date of filing: 04.05.2005
(51) Int. Cl.: A47D 15/00, A61F 5/37

(54) **INFANT SUPPORT**
KLEINKINDSTÜTZE
SUPPORT POUR BEBES

(30) Priority: 16.05.2004 IL 16201204
(43) Date of publication of application: 14.02.2007
(73) Proprietor: Aminach Bedding & Furniture Manufacturing Ltd., 72101 Ramla (IL)
(72) Inventor: JANO, Max, 76804 Mazkeret Batya (IL); REGEV, Itzhak, 69712 Tel Aviv (IL)
(74) Representative: Minoja, Fabrizio
(86) International application number: PCT/IL2005/000470
(87) International publication number: WO 2005/110162

(56) References cited:
- WO-A-86/02814
- CH-A- 557 162
- FR-A1- 2 618 316

## Description

### FIELD OF THE INVENTION

This invention relates to mattresses and beds specially for babies, infants, and children, in particular to reclining mattresses, and to harnesses used in conjunction therewith.

### BACKGROUND OF THE INVENTION

Inclining or propping up a child or infant on a bed has been suggested by the medical profession as being advantageous for the child or infant, both when awake and asleep. Such a posture is particularly helpful during times of illness, particularly colds or sinus infections, where the inclination of the body allows the sinuses to drain freely. Further, in some treatments for gastroesophageal reflux (GER), a child or infant is inclined in the prone position to aid the esophageal passage in remaining firm and tight, and gravity helps to keep the ingested food in the digestive tract.

Various techniques are known for placing the infant or child in the inclined position. In one example, pillows are placed underneath the infant to position the same as required. However, pillows are usually very soft and easily distort as the overlying infant sinks into the pillow, with the potential danger of suffocation should the infant's head turn so that the mouth and nose press against the pillow. Furthermore, pillows do not provide adequate static support, and the infant can easily change position with respect to the pillows, in many cases actually rolling off it.

Another technique involves inclining the infant's mattress, and many conventional devices exist for adjusting the angle of the plane of the mattress within the crib, for example as described in US 5,208,925. However, such mattresses are often difficult to position and adjust, and slings or the like need to be used with these devices, the slings being connected to the side rails or walls of the crib.

In US 4,657,005, a harness is provided having a pair of anchor straps that are pined to one end of the mattress such that they anchor the harness to the higher portion of the inclined mattress, and lateral straps are pined to the mattress to prevent lateral movement or roll when the infant is in the mattress. While the harness allows the infant some mobility of the arms and legs, pinning the anchor straps or the lateral straps to the mattress is not straightforward.

In US 4,989,286 (Re 34,763) a band of bedding material slips over the end of the crib mattress, when this is inclined, to provide an anchor to an infant support sling connected thereto via a relatively narrow neck portion. However, the neck portion allows the infant to pivot and swing with respect to the band.

In US 4,862,535 and US 5,439,008, problems with inclining the mattress are circumvented by providing a wedge-shaped support member that is placed on the mattress in the crib when needed, and the upper surface of the member comprises strips of material that are used for supporting the infant on the member. When it is desired to have the infant lie in the horizontal position, the wedge-shaped member has to be removed, and suitable storage space found therefor.

### SUMMARY OF THE INVENTION

The present invention provides a support system for enabling an infant to be inclined on a surface at a desired angle, and comprises a mattress configured to have a portion thereof pivotably movable with respect to another, stationary portion of the mattress, and an infant harness that is configured to securely support the infant to the movable part.

Thus, the present invention is directed to a support system for an infant or child comprising:
a mattress having a movable part that is pivotable with respect to a stationary part; and
a harness adapted for holding said infant or child, and configured to be releasably secured to said movable part.

While the term "infant" and "child" are used herein interchangeably to refer to human beings of age ranging from very young, including newborn babies, to at least young adult, it also refers to any human being irrespective of age, though in particular those having a height that is within the normal height range between newborn and young adult.

The mattress comprises an external body of resilient cushioning material and an internally disposed support structure, wherein the support structure is adapted for pivotably moving longitudinally opposed portions of the mattress between at least two angular positions. The movable part comprises one said longitudinally opposed portion and said stationary part comprises another one of said longitudinally opposed portions.

The support structure comprises a first frame member and a second frame member, said first frame member supporting said movable portion and said second frame member supporting said stationary portion, wherein said first frame member is pivotably mounted with respect to said second frame member. In one embodiment, the first frame member and the second frame member are each substantially rectangular frames, typically U-shaped tubular frames. The first frame is pivotably mounted with respect to said second frame by means of hinges. In one embodiment, the hinges are adapted to enable a range of discrete angles to be provided between said first frame and said second frame, and the range of discrete angles includes at least one of 0°, about 14°, about 28°, or about 43°, or at least one of 0°, about 10°, about 20°, about 30°, about 40° or higher than 40°, or any other specific desired angle. Alternatively, the hinges are adapted to enable any angle within a range of angles to be provided between said first frame and said second frame, and may include, for example, any angle from about 0° to about 45°.

In one embodiment, the body of resilient cushioning material comprises a first layer and a second layer of resilient cushioning material, wherein the said first layer overlies said second layer and is spaced therefrom to accommodate said support structure therebetween. Preferably, end spacers are provided connecting opposed ends of said first and second layers. Typically, the first and second layers and the spacers are integrally formed, and are comprised of a polyurethane foam, preferably of a fire retardant and hypoallergic polyurethane foam.

Optionally, a third layer is overlaid on said first layer, and typically, the third layer is made from polyethylene foam. Further optionally, a cover is provided for covering said mattress in an enveloping manner.

Alternatively, the body of resilient cushioning material comprises inflatable air-filled or liquid-filled substantially rectangular bags, having a space adapted for accommodating the support structure.

Such a mattress can be used on any bed frame of appropriate size and adjusted to the desired angle without difficulty. Indeed, the stationary part may provide sufficient base support so that the mattress can actually be placed on bed springs, a mattress or a flat surface such as the floor, and where necessary the weight of the infant may be counterbalanced by placing an object of suitable weight on the stationary part, for example. When it is required to have the infant lying in the horizontal position, the movable part is merely lowered again, optionally while the infant is still lying on the mattress. This arrangement also allows the user to test for the optimal angle while the infant is harnessed to the mattress, and avoids having to remove the infant every time the mattress angle is re-adjusted.

The mattress can be of a size suitable to enable a child to sleep thereon, and it is thus possible to use a single mattress for a number of different users from infants to children and/or indeed for any user from infancy up until the user grows too tall to fit on the mattress. Alternatively, the mattress may be of a size for an adult to sleep thereon, and it is thus possible to use a single mattress for a number of different users from infants to adults and/or indeed for any user from infancy up until adulthood or until the user grows too tall to fit on the mattress.

The harness comprises:
a hood comprising a band member and an end member and adapted for being positioned over one end of said mattress when in use; and
a sling adapted for supporting a said infant or child when in use, wherein said sling is mounted in overlying relationship with said band member.

At least a majority of the sling is securely mounted in overlying relationship with a lower portion of said band member, and the end member is connected to an upper portion of said band member. Advantageously, the band member of the hood has a portal or cut-out section intermediate the sling and the end member, i.e., in registry with the expected position of head of the infant that is to be supported by the harness, so that the infant can rest its head directly over the mattress rather than over the hood material itself.

The sling comprises a first sling portion joined to a second sling portion via a crutch portion, wherein said second sling portion when in use is folded over said first sling portion via said crotch portion. The sling is preferably in the form of relatively stiff shell-like portions connected together via the crutch portion, and the shells fold at the crutch portion to provide a cavity for securely receiving the infant. One of the shells is removably or permanently secured to the hood at a number of spaced locations, such that at least a majority of the shell overlies the hood, and such a configuration prevent the harness from pivoting or swinging with respect to the hood.

In one embodiment, the sling comprises a first set of suitable fastener means secured to transversely opposed ends of said first sling portion, and a second set of suitable fastener means secured to transversely opposed ends of the second sling portion, wherein when the second sling portion is folded into overlying relationship with the first sling portion, the first and second sets of fastener means, are brought together enabling the said first and second sling portions to be releasably secured to each other. Preferably, the fastener means of each said set preferably comprises a suitable quick release type fastener, including, for example, any one of hook and loop type fasteners, snap type fasteners, adhesive fasteners, and the like. Alternatively, the fastener means may include any one of zips, buttons, snaps, buckles and the like. Preferably, each set of fastener means are adapted to permit attachment of the second sling portion with respect to the first sling portion at a range of different relative positions to accommodate infants of different sizes.

The hood is configured to fit over the end of the movable part of the mattress, and thus can be fitted or removed from the mattress in a very simple manner. The hood is preferably made from a plastic or nylon material, so that the hood can be easily cleaned in situ without having to remove it from the mattress.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:
**Fig. 1** is a partial isometric view of an embodiment of the system according to the present invention, showing an infant in a face-down position.
**Fig. 2** is a partial isometric view of the embodiment of Fig. 1, showing an infant in a face-up position.
**Fig. 3** is a longitudinal cross-sectional view of a mattress according the embodiment of Figs. 1 and 2.
**Fig. 4** is a transverse side view of some internal components of the mattress of Fig. 3.
**Fig. 5** is a plan view of some internal components of the mattress of Fig.3.
**Fig. 6** is a plan view of the support structure of the mattress of the embodiment of Fig. 3.
**Fig. 7** is a side view of the support structure of the mattress of the embodiment of Fig. 3.
**Fig. 8** is a plan view of the harness of the embodiment of Figs. 1 to 3.

### DETAILED DESCRIPTION OF THE INVENTION

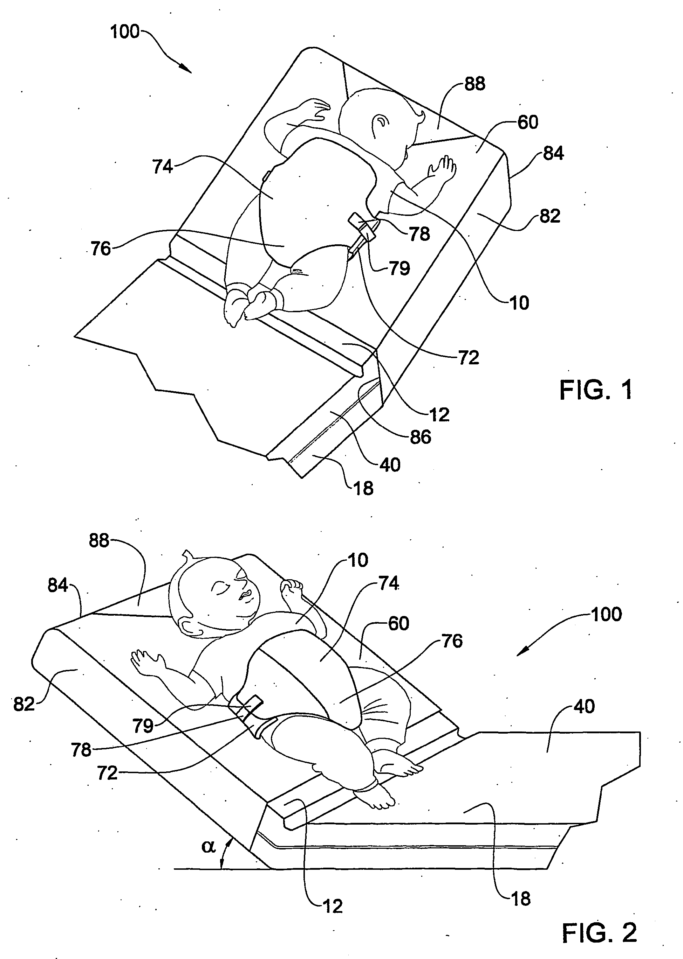

According to the invention, and referring to Figs 1 and 2, an inclinable support system, generally designated with the numeral **100,** is provided for supporting an infant **10** at any one of a range of angles α to the horizontal. The system **100** comprises an inclinable mattress **40,** and a harness **60** that is removably mountable with respect to the mattress **40.**

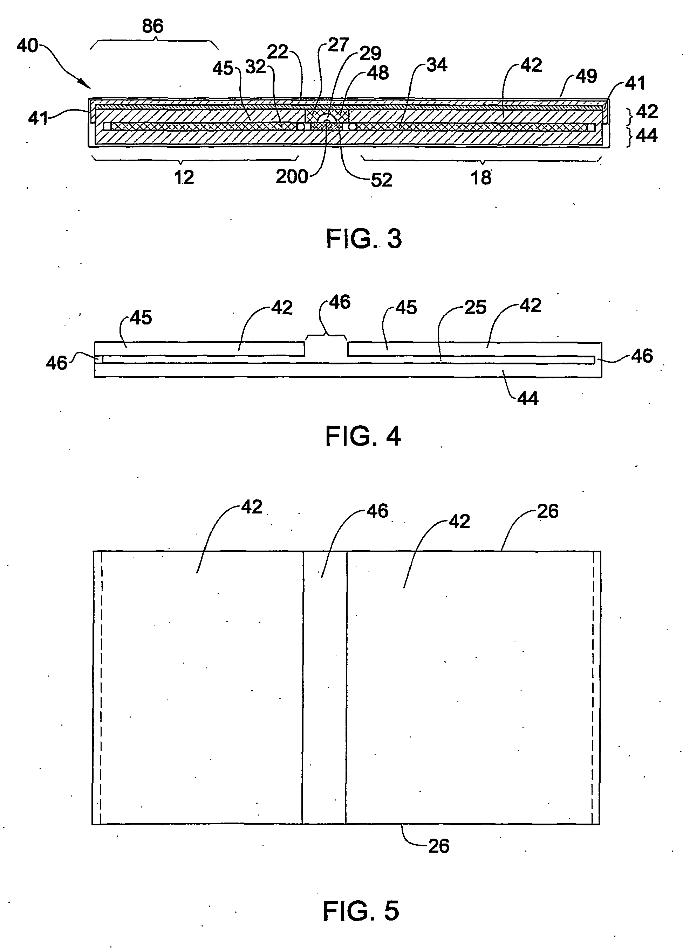

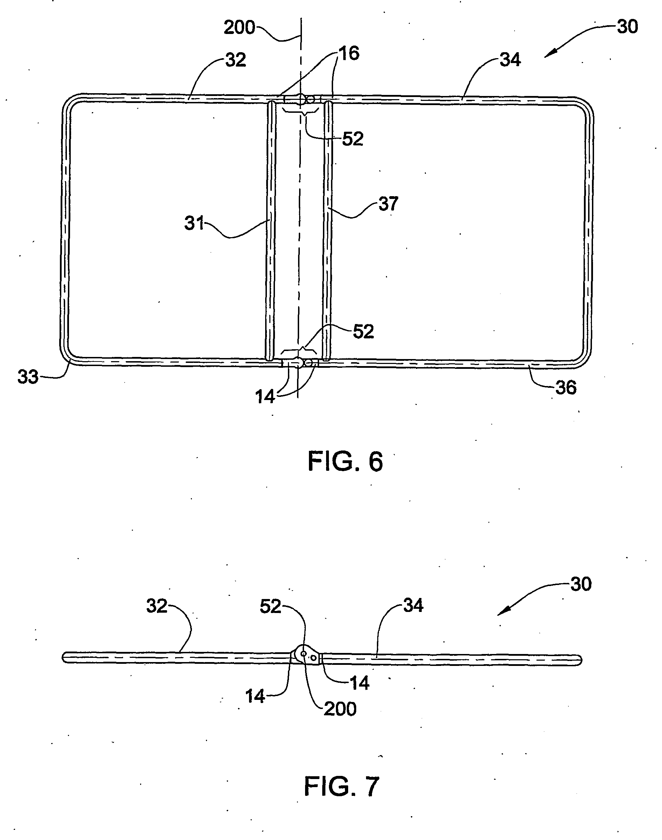

Referring in particular to Figs. 3 to 7, the inclinable mattress **40** is in the form of a pad made from a resilient material and having a cavity **25** housing an internal support structure **30.** The support structure **30** is thus sandwiched between substantially parallel first layer **42** and second layer **44.** The mattress **40** comprises a stationary part or portion **18** and a movable part or portion **12** that is rotatable about a transverse pivot axis **200** with respect to the stationary portion **18** by means of the support structure **30.**

In this embodiment, the support structure **30** is in the form of two substantially rectangular frames **32, 34** pivoted together about pivot axis **200.** Frame **32** is comprised in the movable portion **12,** while frame **34** is comprised in the stationary portion **18.** The frames **32, 34,** are advantageously formed as U-shaped tubular members **33, 36,** respectively, having two pairs of opposed free ends, **14** and **16.** At least one of the frames **32, 34** may comprise at least one cross-brace member, **31** and/or **37** respectively. The frames **32, 34** comprise a pivot mechanism comprising a pair of hinges **52,** one each connecting corresponding opposed free ends **14, 16,** of the members **33, 36.**

In this embodiment, each hinge **52** is configured to enable the frames **32, 34** to be set at predetermined discrete angles α to each other, for example, 0°, 14°, 28° 43°; or 0°, 10°, 20°, 30°, 40° or higher; or indeed any set of specific desired angles, and the hinges **52** each comprise a suitable mechanism for this purpose. For example, each hinge **52** may comprise a pair of facing plates, each plate being rigidly connected to a different one of said frames **32, 34.** For each pair of plates, the plates are biased to press towards each other, and one plate comprises a notch or a rounded protrusion, for example, while the other plate comprises a series of apertures that are circumferentially spaced along an imaginary circle having its center coaxial with axis **200.** The protrusion is configured to fit into each one of the apertures in turn as one frame, and thus one disc, is rotated with respect to the other frame, and thus the other disc, about axis **200.** A predetermined torque is required to separate the plates as the protrusion disengages from one aperture and travels towards the next aperture, and thus the frames are stably positioned at mutual angles that are correlated with the position of these apertures. Such arrangements for hinges **52** are known in the art, and indeed there are many other configurations for hinges **52** that are known in the art and capable of providing the required range of discrete angles between the frames **32, 34.**

Alternatively, each hinge **52** is configured to enable the frames **32, 34** to be set at any desired angle to each other, for example, in the range 0° to 45° or higher, and the hinges **52** each comprise a suitable mechanism for this purpose. For example, each hinge **52** may comprise a pair of facing plates, each plate being rigidly connected to a different one of said frames **32, 34,** and the plates being biased towards each other to provide frictional contact between facing surfaces of the plates. A predetermined torque is required to overcome the static friction between the plates, and when the torque is terminated, the plates, and thus the frames remain stably positioned at the resulting mutual angle. Such arrangements for hinges **52** are also known in the art, and indeed there are many other configurations for hinges **52** that are known in the art and capable of providing the required continuous range of angles between the frames **32, 34.**

The mattress **40** comprises a first layer **42** and second layer **44** of a resilient and preferably somewhat compressible padding material, such as for example polyurethane foam, preferably fire retardant and hypoallergic polyurethane foam. This foam may optionally be supplied in a variety of compression densities. The mattress **40** is substantially rectangular in plan configuration, having a length and width typically approximating those of a child, i.e., larger than those of a cot, though in some variations of this embodiment, the width and length of the mattress **40** may approximate that of a cot. In yet other embodiments, the width and length of the mattress **40** may approximate that of an adult-sized bed. The depth of the mattress **40** is typically generally uniform.

The layers **42, 44** are substantially parallel and spaced one from the other to provide cavity 25, and thus serve to sandwich the support structure **30** therebetween. As best shown in Fig. 3 and Fig. 4, the first layer **42** comprises two sections **45** separated by spacing **46.** Facing longitudinal ends of the first layer **42** and second layer **44** are connected via spacer strips **46.** Preferably, the first layer **42,** second layer **44** and strips **46** are integrally formed.

The movable portion **12** thus comprises one of the sections **45,** strip **46** and part of second layer **44,** and the stationary portion **18** comprises the other one of the sections **45,** the other strip **46** and a part of second layer **44.** In some embodiments, the mattress **40** may be substantially symmetrical about a vertical plane containing the axis **200** and thus either one of the portions **12, 18** may be considered as a movable portion, and the other one as a stationary portion.

A resilient spacer **27** is provided in space **46,** and typically comprises a suitable recess for accommodating the hinges **52** therein and allowing free operation thereof. Preferably, the spacer **27** comprises a transverse arched recess **29** extending from one lateral side **26** of the mattress to the other lateral side **26.** Typically, spacer **27** is more flexible than the first layer **42,** particularly due to the thinner transverse cross-section of the spacer **27** at the apex of the arch thereof, and thus facilitates the bending of mattress **40** about axis **200.**

The mattress **40** optionally further comprises a third layer 48 overlying the first layer **42,** and typically made from a resilient, though harder material than the first layer **42** or second layer **44,** such as for example polyethylene foam.

Optionally, a fourth layer **49** may be provided overlying layer **48,** and having wings **41** extending over the longitudinal ends of the mattress **40.** The fourth layer **49** is typically made from acrylan and is about 1cm thick, to provide added comfort to the user. Optionally, the fourth layer **49** may be provided to over the outside of the second layer **44,** instead of or in addition to the first layer **42.**

Further optionally, the mattress **40** may be fitted with a cover **22,** which may be made from any suitable material, including a textile material, or alternatively a wipable material such as for example nylon or the like. Advantageously, the cover **22** completely envelopes the mattress **40** and comprises a zip or other fastener that enables the cover **22** to be selectively removed when desired.

Optionally, the mattress **40** may be covered with a form fitted bed sheet or other covering.

In one non-limiting example of this embodiment, the second layer **44** is about 1.26m long, about 63cm wide, and about 3cm thick. Sections **45** of the first layer each are about 52cm long, about 63cm wide, and about 3cm thick. Strips **46** are about 1.5cm thick, provide a spacing of about 2cm between the first and second layers, and are about 63cm wide. The third and fourth layers, **48, 49** may be approximately 1cm thick, and about 1.27m long and 63cm wide.

In other embodiments not illustrated herein, the mattress may comprise all the elements as described above, *mutatis mutandis,* but wherein the layers **42, 44,** are replaced with one or more air-filled or liquid-filled substantially rectangular bags, similar in shape and dimensions to the layers **42, 44,** and having a space adapted for accommodating the support structure **30.**

In other embodiments, the support structure **30** may be configured to enable the mattress **40** to pivot in both directions from the horizontal plane. This allows the mattress to be inclined when face up, and also face down, i.e., when the bottom face is turned over so that this is now facing up.

Referring to Figs 1, 2, 3 and 8, the harness **60** comprises a sling **70** mounted onto a hood **80.** The hood **80** comprises a band portion **82** and an end portion **84** which together define a cavity into which an end portion 86 of the movable part **12** of mattress **40** is received as the hood **80** is fitted over this end portion **86.** The end portion 86 is comprised on the movable part **12** and includes at least a part of one of the sections **45,** corresponding portions of the second layer **44,** third layer **48,** and where appropriate also corresponding portions of fourth layer **49** and cover **22.** The hood **80** is made typically from sheet material, preferably a wipable material such as nylon for example, which enables the hood to be kept clean without the need for removing it from the mattress. Alternatively, the hood **80** may be made from a fabric or pulp material. Alternatively, the hood **80** may be made from a disposable material, and thus hood **80** is removable from the sling **60** when desired or soiled, to be replaced by a new hood **80.**

Optionally, a head rest (not shown) may be provided on the harness for stabilizing the head of an infant.

Optionally, the hood **80** comprises an opening or portal **88** that enables the infant's head to rest directly against the mattress material when the hood **80** is engaged over the movable part 12. Additionally or alternatively, the hood may be adapted to enable a wipe, towel or the like to be secured to the hood such that the infant's head rests on the wipe or towel rather than directly on the hood.

The sling **70** is configured to encircle and support the torso of the infant, while allowing free movement of the arms, legs and head thereof. The sling **70** comprises a relatively stiff though foldable sheet material and includes a first portion **72,** and a second portion **74** which in use is folded over the first portion **72** via a crotch portion **76.** The first sling portion **72** and the second sling portion **74** may be shell-shaped, having cavities which are generally complementary to the rounded shape of infant's torso. A first set of suitable fastener means **78** are secured to transversely opposed ends of the first portion **72,** and a second set of suitable fastener means **79** are secured to transversely opposed ends of the second portion **74.** When the second portion **74** is folded into overlying relationship with the first portion **72,** the first and second sets of fastener means, **78, 79** are brought together, enabling the sling portions **72, 74** to be releasably secured to each other. The fastener means **78, 79** of each set preferably comprise any suitable quick release type fastener, such as for example hook and loop (e.g., Velcro) type fasteners, snap type fasteners, adhesive fasteners, and so on. Alternatively, other types of fasteners may be used, including for example, zips, buttons, buckles and so on. Preferably, each set of fastener means **78, 79** permit attachment of the second sling portion **74** with respect to the first sling portion **72** at a range of different relative positions to accommodate infants of different sizes.

The sling material is preferably a washable or otherwise cleanable material, preferably comprising a lining of water resistant fabric or material for enabling the infant to be cleaned up with some ease.

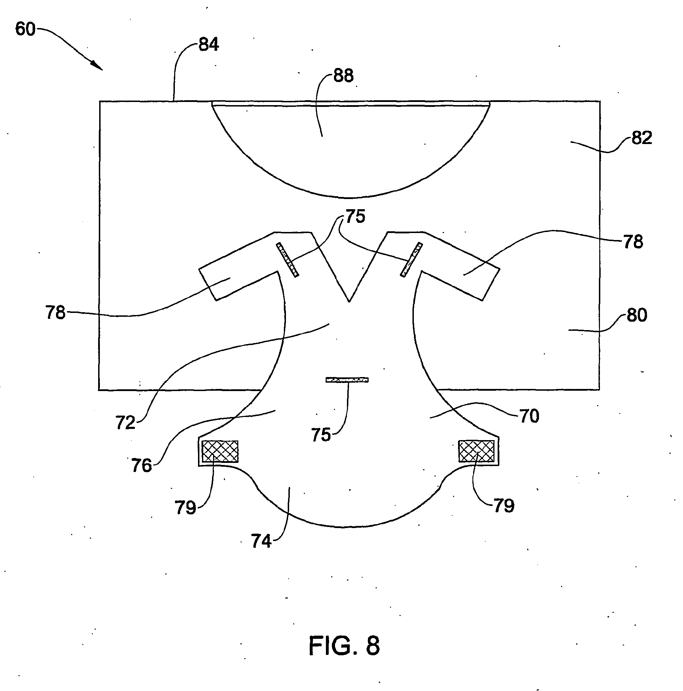

The sling **70** is mounted to the lower part of the hood **80** in such a manner such that when the second sling portion **74** is folded over the first sling portion **72,** the second sling portion **74** overlies the lower part of hood **80.** The first portion **72** may be permanently joined to the hood **80** via stitching **75,** or alternatively releasably joined using any suitable fasteners, including buckles, Velcro, buttons, adhesive strips, zips and so on. In any case, the locations at which the first portion **72** is secured to the hood are such, for example near the crotch part and at the upper extremities, as illustrated in Fig. 8, that at least a majority of the first part **72** is in overlying and secured relationship with respect to the hood **80.** This provides stability to the infant in the sling **70,** which is then restricted in swinging or pivoting movements with respect to the hood **80.**

As illustrated in Fig. 1, the infant's chest and abdomen may be placed against the first sling portion **72,** and the infant's back against the second sling portion **74,** so that the infant is face-up. Alternatively, and as illustrated in Fig. 2, the infant's chest and abdomen may be placed against the second sling portion **74,** and the infant's back against the first sling portion **72,** so that the infant is face-down.

In other embodiments, the sling **70** may comprises a different arrangement for securing the infant to the hood **80.** For example, the sling may comprise a seat having apertures for the legs of the infant to be inserted therethrough, and suitable straps or the like to restrain the infant on the seat. Such a seat is securely fastened, permanently or removably, to the hood.

The system **100** may be operated in a number of different ways. In one exemplary way, the mattress **40** is placed over bed springs or a box spring of a bed, and the stationary part **18** is secured on the bed by any suitable means, including fasteners, belts, hook-and-loop type fasteners and so on, or, a suitable weight is placed on the stationary part **18** to counterbalance the weight of the infant. Alternatively, the mattress **40** is placed on a regular mattress which is on the bed, or directly on the floor, for example, and the counterbalance weight is placed on the stationary part **18.** Then, the movable part **12** is rotated with respect to the stationary part **18** to the desired inclination angle α via hinges **52.** The hood **80** is placed over the end **86** of the movable part **12** so that the sling **70** is on the upper portion of the movable part **12.** The sling **70** is then opened by unfastening the pairs of fastener means **78, 79,** and the second sling portion **74** unfolded with respect to the first sling portion 72. The infant is then held against the first portion **72,** face up as in Fig. 1, or face down as in Fig. 2, and the second sling portion **74** is folded about the crutch portion **76** and secured to the first sling portion **72** via the pairs of fastener means **78, 79.** Removing the infant is by reversing the above steps. When face down, the infant's face is lying directly on the mattress **40** via portal **88,** which is more comfortable than the material of the hood **80,** which is typically made from a plastic or nylon-type wipable material. Thus, should the infant experience reflux and vomit, this can be easily removed from the hood **80** without having to dismantle and wash the same. Optionally, a towel or the like may be placed between the infant's head and the hood **80** or movable part **12.**

Alternatively, the infant may be secured to the sling **70** while the mattress **40** is horizontal, and the movable part **12** subsequently rotated to the desired angle α. Alternatively, the pairs of fastener means **78, 79** may allow the infant to be inserted into the sling **70** after the first sling portion **72** is secured with the second sling portion. For this purpose, each set of fastener means **78, 79** permit attachment of the second sling portion **74** with respect to the first sling portion **72** at a range of different relative positions, and can be set at a wide position to receive the infant, and then tightened to secure the infant in place.

The mattress **40** comprises a relatively hard layer **48,** which is advantageous for an infant or baby up to 9 months old or so, for example. Thereafter, or when there is less need for inclining the mattress **40,** the mattress **40** may be turned over so that the bottom thereof, comprising a relatively softer second layer **44,** is now face up, affording the infant or child or another user a more comfortable and softer surface.

Thus the support system of the invention is multi-functional, allowing an infant or child to be inclined in a user friendly manner whenever required or desired, and also allowing the same mattress to be used when the infant is older, or indeed for other users who only require a regular horizontal mattress.

While there has been shown and disclosed exemplary embodiments in accordance with the invention, it will be appreciated that many changes may be made therein without departing from the scope of the claims.

## Claims

1. A support system (100) for an infant or child (10) comprising:
a mattress (40) having a movable part (12) that is pivotable with respect to a stationary part (18) of the mattress and
a harness (60) adapted for holding said infant or child, and being releasably secured to said movable part (12) of the mattress.

2. A support system according to claim 1, wherein said mattress comprises an external body of resilient cushioning material and an internally disposed support structure (30), wherein the support structure is adapted for pivotably moving longitudinally opposed portions of the mattress between at least two angular positions.

3. A support system according to claim 2, wherein said movable part (12) comprises one said longitudinally opposed portion and said stationary part (18) comprises another one of said longitudinally opposed portions.

4. A support system according to claim 2, wherein said support structure comprises a first frame member (32) and a second frame member (34), said first frame member supporting said movable portion and said second frame member supporting said stationary portion, wherein said first frame member is pivotably mounted with respect to said second frame member.

5. A support system according to claim 4 wherein said first frame member (32) and said second frame member (34) are each substantially rectangular frames.

6. A support system according to claim 4, wherein said first frame is pivotably mounted with respect to said second frame by means of hinges (52).

7. A support system according to claim 6, wherein said hinges (52) are adapted to enable a range of discrete angles to be provided between said first frame and said second frame.

8. A support system according to claim 7, wherein said range of discrete angles includes at least one of 0°, about 14°, about 28°, or about 43°.

9. A support system according to claim 7, wherein said range of discrete angles includes at least one of 0°, about 10°, about 24°, about 30°, about 40° or higher than 40°, or any other specific desired angle.

10. A support system according to claim 6, wherein said hinges (52) are adapted to enable any angle within a range of angles to be provided between said first frame and said second frame.

11. A support system according to claim 10, wherein said range of angles includes any angle from about 0° to about 45°.

12. A support system according to claim 2, wherein said body of resilient cushioning material comprises a first layer (42) and a second layer (44) of resilient cushioning material, wherein the said first layer overlies said second layer and is spaced therefrom to accommodate said support structure (30) therebetween.

13. A support system according to claim 12, further comprising end spacers (46) connecting opposed ends of said first and second layers.

14. A support system according to claim 13, wherein said first and second layers are comprised of a polyurethane foam.

15. A support system according to claim 14, wherein said first and second layers are comprised of a fire retardant and hypoallergic polyurethane foam

16. A support system according to claim 12, further comprising a third layer (48) overlaid on said first layer (42).

17. A support system according to claim 16, wherein said third layer (48) is made from polyethylene foam.

18. A support system according to claim 16, further comprising a cover (22) for covering said mattress in an enveloping manner.

19. A support system according to claim 1, wherein said harness (60) comprises;
a hood (80) comprising a band member (82) and an end member (84) and adapted for being positioned over one end of said mattress when in use; and
a sling (70) adapted for supporting a said infant or child when in use, wherein said sling is mounted in overlying relationship with said band member (82).

20. A support system according to claim 19, wherein at least a majority of said sling (70) is securely mounted in overlying relationship with a lower portion of said band member (82), and wherein said end member (84) is connected to an upper portion of said band member (82).

21. A support system according to claim 19, wherein said band member (82) comprises a portal (88) intermediate said harness and said end member (84).

22. A support system according to claim 19, wherein said sling (70) comprises a first sling portion (72) joined to a second sling portion (74) via a crutch portion (76), wherein said second sling portion (74) when in use is folded over said first sling portion (72) via said crotch portion (76).

23. A support system according to claim 22, further comprising a first set of suitable fastener means (78) secured to transversely opposed ends of said first sling portion, (72) and a second set of suitable fastener means (79) secured to transversely opposed ends of the second sling portion (74), wherein when the second sling portion is folded into overlying relationship with the first sling portion, the first and second sets of fastener means, are brought together enabling said first and second sling portions to be releasably secured to each other.

24. A support system according to claim 23, wherein the said fastener means (78,79) of each said set preferably comprise a suitable quick release type fastener.

25. A support system according to claim 24, wherein said fastener means (78,79) include any one of hook and loop type fasteners, snap type fasteners, adhesive fasteners, and the like.

26. A support system according to claim 23, wherein said fastener means (78,79) include any one of zips, buttons, snaps, buckles and the like.

27. A support system according to claim 23, wherein each set of fastener means (78,79) are adapted to permit attachment of the second sling portion (74) with respect to the first sling portion (72) at a range of different relative positions to accommodate infants of different sizes.

## Patentansprüche

1. Tragesystem (100) für einen Säugling oder ein Kind (10), umfassend:
eine Matratze (40) mit einem beweglichen Teil (12), der in bezug auf einen unbeweglichen Teil (18) der Matratze drehbar ist; und
eine Halterung (60), die zum Halten des Säuglings oder Kindes angepaßt ist und lösbar mit dem beweglichen Teil (12) der Matratze gesichert ist.

2. Tragesystem nach Anspruch 1, wobei die Matratze einen äußeren Grundkörper aus elastischem Polstermaterial und eine innen angeordnete tragende Struktur (30) umfaßt, wobei die tragende Struktur so angepaßt ist, daß in Längsrichtung entgegengesetzte Abschnitte der Matratze zwischen mindestens zwei Winkelpositionen drehbar bewegt werden können.

3. Tragesystem nach Anspruch 2, wobei der bewegliche Teil (12) einen in Längsrichtung entgegengesetzten Abschnitt umfaßt und der unbewegliche Teil (18) einen anderen der in Längsrichtung entgegengesetzten Abschnitte umfaßt.

4. Tragesystem nach Anspruch 2, wobei die tragende Struktur ein erstes Rahmenelement (32) und ein zweites Rahmenelement (34) umfaßt, wobei das erste Rahmenelement den beweglichen Abschnitt trägt und das zweite Rahmenelement den unbeweglichen Abschnitt trägt, wobei das erste Rahmenelement in bezug auf das zweite Rahmenelement drehbar angebracht ist.

5. Tragesystem nach Anspruch 4, wobei das erste Rahmenelement (32) und das zweite Rahmenelement (34) jeweils im wesentlichen rechtwinklige Rahmen sind.

6. Tragesystem nach Anspruch 4, wobei der erste Rahmen in bezug auf den zweiten Rahmen mittels Gelenken (52) drehbar angebracht ist.

7. Tragesystem nach Anspruch 6, wobei die Gelenke (52) so angepaßt sind, daß ein Bereich einzelner Winkel zwischen dem ersten Rahmen und dem zweiten Rahmen bereitgestellt werden kann.

8. Tragesystem nach Anspruch 7, wobei der Bereich einzelner Winkel mindestens einen von 0°, etwa 14°, etwa 28° oder etwa 43° umfaßt.

9. Tragesystem nach Anspruch 7, wobei der Bereich einzelner Winkel mindestens einen von 0°, etwa 10°, etwa 20°, etwa 30°, etwa 40° oder höher als 40° oder irgendeinen anderen speziellen gewünschten Winkel umfaßt.

10. Tragesystem nach Anspruch 6, wobei die Gelenke (52) so angepaßt sind, daß irgendein Winkel innerhalb eines Winkelbereiches zwischen dem ersten Rahmen und dem zweiten Rahmen bereitgestellt werden kann.

11. Tragesystem nach Anspruch 10, wobei der Winkelbereich irgendeinen Winkel von etwa 0° bis etwa 45° umfaßt.

12. Tragesystem nach Anspruch 2, wobei der Grundkörper aus elastischem Polstermaterial eine erste Schicht (42) und eine zweite Schicht (44) aus elastischem Polstermaterial umfaßt, wobei die erste Schicht die zweite Schicht überlagert und davon beabstandet ist, um die tragende Struktur (30) dazwischen unterzubringen.

13. Tragesystem nach Anspruch 12, ferner umfassend Abstandshalter am Ende (46), die die entgegengesetzten Enden der ersten und zweiten Schicht verbinden.

14. Tragesystem nach Anspruch 13, wobei die erste und zweite Schicht aus einem Polyurethanschaum bestehen.

15. Tragesystem nach Anspruch 14, wobei die erste und zweite Schicht aus einem Feuerschutz- und hypoallergischen Polyurethanschaum bestehen.

16. Tragesystem nach Anspruch 12, ferner umfassend eine dritte Schicht (48) über der ersten Schicht (42).

17. Tragesystem nach Anspruch 16, wobei die dritte Schicht (48) aus Polyethylenschaum besteht.

18. Tragesystem nach Anspruch 16, ferner umfassend eine Abdeckung zum Abdecken der Matratze in einer umhüllenden Weise.

19. Tragesystem nach Anspruch 1, wobei die Halterung (60):
eine Haube (80), die ein Bandelement (82) und ein Endelement (84) umfaßt und so angepaßt ist, daß sie bei Verwendung über einem Ende der Matratze positioniert werden kann; und
ein Tragetuch (70), das so angepaßt ist, daß es bei Verwendung den Säugling oder das Kind tragen kann, wobei das Tragetuch in überlagernder Beziehung zu dem Bandelement (82) angebracht ist, umfaßt.

20. Tragesystem nach Anspruch 19, wobei zumindest ein Großteil des Tragetuches (70) sicher in überlagernder Beziehung zu einem unteren Abschnitt des Bandelements (82) angebracht ist, und wobei das Endelement (84) mit einem oberen Abschnitt des Bandelements (82) verbunden ist.

21. Tragesystem nach Anspruch 19, wobei das Bandelement (82) einen Eingang (88) zwischen der Halterung und dem Endelement (84) umfaßt.

22. Tragesystem nach Anspruch 19, wobei das Tragetuch (70) einen ersten Tragetuchabschnitt (72), der mit einem zweiten Tragetuchabschnitt (74) mittels eines Stützabschnittes (76) verbunden ist, umfaßt, wobei der zweite Tragetuchabschnitt (74) bei der Verwendung über den ersten Tragetuchabschnitt (72) mittels des Stützabschnittes (76) gefaltet wird.

23. Tragesystem nach Anspruch 22, ferner umfassend ein erstes Set geeigneter Befestigungsmittel (78), das an quer entgegengesetzte Enden des ersten Tragetuchabschnittes (72) gesichert ist, und ein zweites Set geeigneter Befestigungsmittel (79), das an quer entgegengesetzte Enden des zweiten Tragetuchabschnittes (74) gesichert ist, wobei der zweite Tragetuchabschnitt über den ersten Tragetuchabschnitt gefaltet wird, das erste und zweite Set Befestigungsmittel zusammengebracht werden, wodurch der erste und zweite Tragetuchabschnitt lösbar aneinander gesichert werden können.

24. Tragesystem nach Anspruch 23, wobei die Befestigungsmittel (78, 79) jedes Sets vorzugsweise eine geeignete schnell lösbare Befestigung umfassen.

25. Tragesystem nach Anspruch 24, wobei die Befestigungsmittel (78, 79) irgendwelche Haken- und Schlaufenbefestigungen, Schnappbefestigungen, Klebebefestigungen und dergleichen umfassen.

26. Tragesystem nach Anspruch 23, wobei die Befestigungsmittel (78, 79) irgendeines von Reißverschlüssen, Knöpfen, Druckknöpfen, Schnallen und dergleichen umfassen.

27. Tragesystem nach Anspruch 23, wobei jedes Set von Befestigungsmitteln (78, 79) so angepaßt ist, daß der zweite Tragetuchabschnitt (74) in bezug auf den ersten Tragetuchabschnitt (72) in einem Bereich verschiedener relativer Positionen, um Säuglinge verschiedener Größen unterzubringen, verbunden werden kann.

## Revendications

1. Système de support (100) pour un bébé ou un enfant (10), comprenant :
un matelas (40) ayant une partie mobile (12) qui peut pivoter par rapport à une partie fixe (18) du matelas ; et
un harnais (60) adapté pour maintenir ledit bébé ou enfant et étant fixé de manière amovible à ladite partie mobile (12) du matelas.

2. Système de support selon la revendication 1, dans lequel ledit matelas comprend un corps externe en matériau amortissant élastique et une structure de support (30) disposée intérieurement, dans lequel la structure de support est adaptée pour déplacer de manière pivotante des parties longitudinalement opposées du matelas entre au moins deux positions angulaires.

3. Système de support selon la revendication 2, dans lequel ladite partie mobile (12) comprend ladite une partie longitudinalement opposée et ladite partie fixe (18) comprend l'autre parmi lesdites parties longitudinalement opposées.

4. Système de support selon la revendication 2, dans lequel ladite structure de support comprend un premier élément de châssis (32) et un second élément de châssis (34), ledit premier élément de châssis supportant ladite partie mobile et ledit second élément de châssis supportant ladite partie fixe, dans lequel ledit premier élément de châssis est monté de manière pivotante par rapport audit second élément de châssis.

5. Système de support selon la revendication 4, dans lequel ledit premier élément de châssis (32) et ledit second élément de châssis (34) sont chacun des châssis sensiblement rectangulaires.

6. Système de support selon la revendication 4, dans lequel ledit premier châssis est monté de manière pivotante par rapport audit second châssis au moyen d'articulations (52).

7. Système de support selon la revendication 6, dans lequel lesdites articulations (52) sont adaptées pour permettre de proposer une plage d'angles distincts entre ledit premier châssis et ledit second châssis.

8. Système de support selon la revendication 7, dans lequel ladite plage d'angles distincts comprend au moins l'un parmi 0°, environ 14°, environ 28° ou environ 43°.

9. Système de support selon la revendication 7, dans lequel ladite plage d'angles distincts comprend au moins l'un parmi 0°, environ 10°, environ 20°, environ 30°, environ 40° ou supérieur à 40°, ou n'importe quel autre angle souhaité spécifique.

10. Système de support selon la revendication 6, dans lequel lesdites articulations (52) sont adaptées pour permettre de proposer n'importe quel angle dans une plage d'angles distincts entre ledit premier châssis et ledit second châssis.

11. Système de support selon la revendication 10, dans lequel ladite plage d'angles comprend n'importe quel angle d'environ 0° à environ 45°.

12. Système de support selon la revendication 2, dans lequel ledit corps de matériau d'amortissement élastique comprend une première couche (42) et une seconde couche (44) de matériau amortisseur élastique, dans lequel ladite première couche recouvre ladite seconde couche et est espacée de celle-ci pour loger ladite structure de support (30) entre elles.

13. Système de support selon la revendication 12, comprenant en outre des entretoises d'extrémité (46) raccordant les extrémités opposées desdites première et seconde couches.

14. Système de support selon la revendication 13, dans lequel lesdites première et seconde couches sont composées d'une mousse de polyuréthane.

15. Système de support selon la revendication 14, dans lequel lesdites première et seconde couches sont composées d'une mousse de polyuréthane ignifuge et hypoallergénique.

16. Système de support selon la revendication 12, comprenant en outre une troisième couche (48) appliquée sur ladite première couche (42).

17. Système de support selon la revendication 16, dans lequel ladite troisième couche (48) est composée d'une mousse de polyéthylène.

18. Système de support selon la revendication 16, comprenant en outre un couvercle (22) pour recouvrir ledit matelas d'une manière enveloppante.

19. Système de support selon la revendication 1, dans lequel ledit harnais (60) comprend :
un capuchon (80) comprenant un élément de bande (82) et un élément d'extrémité (84) et adapté pour être positionné sur une extrémité dudit matelas lorsqu'il est utilisé ; et
un porte-bébé (70) adapté pour supporter ledit bébé ou l'enfant lorsqu'il est utilisé, dans lequel ledit porte-bébé est monté en relation de superposition avec ledit élément de bande (82).

20. Système de support selon la revendication 19, dans lequel au moins une majorité dudit porte-bébé (70) est montée de manière fixe en relation de superposition avec une partie inférieure dudit élément de bande (82), et dans lequel ledit élément d'extrémité (84) est raccordé à une partie supérieure dudit élément de bande (82).

21. Système de support selon la revendication 19, dans lequel ledit élément de bande (82) comprend une entrée (88) entre ledit harnais et ledit élément d'extrémité (84).

22. Système de support selon la revendication 19, dans lequel ledit porte-bébé (70) comprend une première partie de porte-bébé (72) assemblée à une seconde partie de porte-bébé (74) via une partie d'entrejambes (76), dans lequel ladite seconde partie de porte-bébé (74) lorsqu'il est utilisé, est repliée sur ladite première partie de porte-bébé (72) via ladite partie d'entrejambes (76).

23. Système de support selon la revendication 22, comprenant en outre un premier ensemble de moyens de fixation appropriés (78) fixé aux extrémités transversalement opposées de ladite première partie de porte-bébé (72) et un second ensemble de moyens de fixation appropriés (79) fixé aux extrémités transversalement opposées de la seconde partie de porte-bébé (74), dans lequel lorsque la seconde partie de porte-bébé est pliée en relation superposée avec la première partie de porte-bébé, des premier et second ensembles de moyens de fixation sont rassemblés, permettant auxdites première et seconde parties de porte-bébé d'être fixées de manière détachable entre elles.

24. Système de support selon la revendication 23, dans lequel lesdits moyens de fixation (78, 79) de chacun desdits ensembles comprennent de préférence une fixation de type à libération rapide appropriée.

25. Système de support selon la revendication 24, dans lequel lesdits moyens de fixation (78, 79) comprennent l'un quelconque parmi les fixations de type Velcro, les fixations de type à pressions, les fixations adhésives, et similaires.

26. Système de support selon la revendication 23, dans lequel lesdits moyens de fixation (78, 79) comprennent l'un quelconque parmi les fermetures éclair, les boutons, les pressions, les boucles et similaires.

27. Système de support selon la revendication 23, dans lequel chaque ensemble de moyens de fixation (78, 79) est adapté pour permettre la fixation de la seconde partie de porte-bébé (74) par rapport à la première partie de porte-bébé (72) selon une plage de positions relatives différentes pour accepter des bébés de différentes tailles.
